Europäisches Patentamt

European Patent Office

Office européen des brevets

(11) Veröffentlichungsnummer: **0 488 089 A1**

# EUROPÄISCHE PATENTANMELDUNG

(21) Anmeldenummer: **91120005.3**

(22) Anmeldetag: **23.11.91**

(51) Int. Cl.5: **A61K 31/195**, A61K 9/12,
A61K 47/00, A61K 47/26,
A61K 47/20

(30) Priorität: **30.11.90 DE 9016291 U**

(43) Veröffentlichungstag der Anmeldung:
**03.06.92 Patentblatt 92/23**

(84) Benannte Vertragsstaaten:
**AT BE CH DE DK ES FR GB IT LI LU NL SE**

(71) Anmelder: **Kali-Chemie Pharma GmbH**
**Hans-Böckler-Allee 20 Postfach 220**
**W-3000 Hannover 1(DE)**

(72) Erfinder: **Klimek, Rainhardt**
**Thönser Trift 2**
**W-3006 Burgwedel(DE)**
Erfinder: **Bonnacker, Ingo**
**Sedanstrasse 6**
**W-3250 Hameln(DE)**

(74) Vertreter: **Lauer, Dieter, Dr.**
**c/o Solvay Deutschland GmbH, Postfach 220,**
**Hans-Böckler-Allee 20**
**W-3000 Hannover 1(DE)**

(54) **Topisch applizierbare Diclofenac-Zubereitungen.**

(57) Beschrieben wird eine neue Diclofenac-Zubereitung zur topischen Applikation, die zusammen mit einem Treibgas in einem Druckgasbehälter abgefüllt wird und aus diesem über einen Zerstäuber mit Hilfe des Treibgases verschäumt und als Diclofenac enthaltender Schaum abgegeben werden kann.

EP 0 488 089 A1

Die vorliegende Erfindung betrifft Diclofenac-Zubereitungen für die topische Applikation.

Diclofenac (2-(2,6-Dichlor-anilino)-phenyl-)essigsäure) ist ein seit langem bewährter Arzneiwirkstoff, der hauptsächlich als nichtsteroidales Antirheumatikum oder Antiphlogistikum angewendet wird.

Diclofenac wurde bisher für die topische Applikation in Form von Gelen eingesetzt.

Der vorliegenden Erfindung lag die Aufgabe zugrunde, topisch applizierbare, antirheumatisch wirksame Diclofenac-Zubereitungen zur Verfügung zu stellen, die sich leicht handhaben lassen und eine verbesserte Verteilbarkeit auf der Haut aufweisen.

Es wurde nun eine neue topisch applizierbare Diclofenac-Zusammensetzung gefunden, welche sich durch eine gute Verteilbarkeit auf der Haut und eine gute Verschäumbarkeit auszeichnet.

Es wurde gefunden, daß die erfingundsgemäße neue Diclofenac-Zusammensetzung zusammen mit einem Treibgas in einen Druckgasbehälter abgefüllt werden und aus diesem über einen Zerstäuber mit Hilfe des Treibgases verschäumt und als Diclofenac enthaltender Schaum zur topischen Applikation abgegeben werden kann.

Die vorliegende Erfindung betrifft daher topisch applizierbare Diclofenac-haltige Emulsionen, dadurch gekennzeichnet, daß sie 0,5 bis 3 Gew.-% eines Diclofenac-Salzes, 10 bis 20 Gew.-% eines flüssigen Öles aus der Gruppe dünnflüssiger Paraffinöle und flüssiger Triglyceride von Fettsäuren mit 8 bis 18 Kohlenstoffatomen, 6 bis 14 Gew.-% eines Tensid-Gemisches aus ionischen Tensiden aus der Gruppe der Alkylsulfate und nichtionischen Tensiden aus der Gruppe der Sorbitanfettsäureester und der Gruppe der Polyoxyethylensorbitanfettsäureester und 1 bis 3 Gew.-% eines aliphatischen Alkohols mit 16 bis 18 Kohlenstoffatomen und Wasser enthalten.

Ferner betrifft die Erfindung Diclofenac-haltige Anordnungen, dadurch gekennzeichnet, daß sie die vorstehende Diclofenac-haltigen Emulsion und Treibgas in einem mit einen Zerstäuber versehenen Druckgasbehälter angeordnet enthalten.

In den erfindungsgemäßen Zubereitungen ist Diclofenac in der verschäumbaren Mischung in Form physiologisch verträglicher Salze, vorzugsweise als Natrium-Salz, in Wasser gelöst enthalten. Da Diclofenac-Salze in Wasser nur bedingt löslich sind, müssen relativ verdünnte Lösungen eingesetzt werden. Geeignete Diclofenac-Salzkonzentrationen liegen, bezogen auf das Gesamtgewicht der Emulsion, z.B. zwischen 0,5 bis 3,0 Gew.%, beispielsweise 1 Gew.-%.

Die verschäumbare Diclofenac-Zubereitung ist eine Ölin-Wasser-Emulsion. Da nur sehr verdünnte Diclofenac-Lösungen verwendet werden, ist der Anteil der wäßrigen Phase in der Emulsion relativ hoch. Als Ölphase wird dünnflüssiges Paraffinöl (Paraffinum perliquidum) verwendet. Es können auch flüssige Triglyceride von Fettsäuren mit 8 bis 18 Kohlenstoffatomen, beispielsweise mittelkettige Triglyceride von gesättigten Fettsäuren mit 8 bis 12 Kohlenstoffatomen wie z.B. Miglyol[R] 812 Neutralöl verwendet werden. Es bestehen 10 bis 20 Gew.-%, zweckmäßigerweise ca. 15 Gew.-% der Emulsion aus flüssigem Öl, insbesondere aus dünnflüssigem Paraffinöl.

Als Emulgatoren werden Gemische ionischer und nichtionischer Tenside in einem Konzentrationsbereich von 6 bis 14 Gew.-% verwendet. Die ionischen Tenside gehören zur Gruppe der Alkylsulfate mit Alkylresten von 11 bis 17 Kohlenstoffatomen wie z.B. Natriumdodecylsulfat. Als nichtionische Tenside werden Gemische aus Sorbitanfettsäureestern und Polyoxyethylensorbitanfettsäureestern eingesetzt. Als Sorbitanfettsäureester werden Ester des nichtsubstituierten Sorbitans mit 1 bis 3 Fettsäuren, insbesondere Fettsäuren mit 12 bis 18 Kohlenstoffatomen, bezeichnet. Zu der Gruppe der Sorbitanfettsäureester gehören beispielsweise Monofettsäureester wie Sorbitanmonosterarat, Difettsäureester wie Sorbitandistearat oder -dipalmitat und Trifettsäureester wie Sorbitantristearat. Aus der Gruppe der Polyoxyethylensorbitanfettsäureester eignen sich Mono-, Di und Triester von Polyoxyethylensorbitan mit bis zu 80, insbesondere bis zu 40 Oxyethyleneinheiten mit Fettsäuren mit 12 bis 18 Kohlenstoffatomen. Beispiele für Polyoxyethylensorbitanfettsäureester sind Polyoxyethylen(40)-sorbitanmonopalmitat oder Polyoxyethylen(20)-sorbitanmonostearat. Zweckmäßigerweise stehen die ionischen Tenside zu den nichtionischen Tensiden in einem Mischungsverhältnis von 1:10 bis 1:20. Bevorzugt enthalten die Emulsionen, bezogen auf das Gesamtgewicht an Emulsion 0,25 bis 0,75 Gew.-% Natriumdodecylsulfat, 5 bis 10 Gew.-% Polyoxyethylen(20)-sorbitanmonostearat und 1,5 bis 3,0 Gew.-% Sorbitanmonostearat.

Um dem zu bildenden, sehr wasserhaltigen Schaum eine höhere Festigkeit zu verleihen, enthält die erfindungsgemäße Emulsion 1,5 bis 3 Gew.-% eines aliphatischen Alkohol mit 16 bis 18 Kohlenstoffatomen wie z.B. Cetylalkohol und/oder Stearylalkohol. Vorzugsweise verwendet man Cetylalkohol. Auch ein Gemisch aus Cetylalkohol und Stearylalkohol im Verhältnis 1:5 bis 5:1, beispielsweise 1:1, kann verwendet werden.

Die Diclofenac-haltige Emulsion wird hergestellt, indem man eine wäßrige Diclofenac-Salzlösung, enthaltend 0,5 bis 3 Gew.-% Diclofenac-Salz bezogen auf das Gesamtgewicht der Emulsion, mit den übrigen Bestandteilen emulgiert. Die erhaltene Emulsion kann zusammen mit einem Treibgas in einem mit einem Zerstäuber versehenen Druckgasbehälter abgefüllt werden.

Als Treibmittel können alle zur Versprühung von schaumbildenden Aerosolen üblichen Treibgase und Treibgasgemische wie z.B. Distickstoffoxid, Propan/Butan oder gasförmige Halogenkohlenwasserstoffe oder Gemische der vorgenannten Substanzen in den bei Druckgaspackungen üblichen Konzentrationen, beispielsweise Konzentrationen von 6 bis 8 Gew.-%, bezogen auf die Emulsion, verwendet werden. Unter gasförmigen Halogenkohlenwasserstoffen werden Fluorchlorkohlenwasserstoffe, z.B. Chlordifluormethan oder 1,1-Dichlor-1-Fluorethan, oder vorzugsweise Fluorkohlenwasserstoffe, z.B. 1,1,1-Trifluorethan oder 1,1,1,1-Tetrafluorethan, verstanden. Als Treibgase bevorzugt sind Distickstoffoxid und/oder Propan/Butan.

Die erfindungsgemäßen Emulsionen können in für Sprays übliche Druckgaspackungen, die zur wiederholten Entnahme mit Zerstäubern, z.B. allgemein gebräuchlichen Sprühaufsätzen oder Dosierköpfen zur Schaumversprühung ausgestattet sind, abgefüllt werden.

Ein großer Vorteil der erfindungsgemäßen Diclofenac-Zubereitungen liegt in der einfachen Handhabung. Durch Betätigung des Sprühaufsatzes kann die erfindungsgemäße Zubereitung zu einem pharmazeutisch wirksamen Schaum verschäumt werden, der problemlos am gewünschten Applikationsort dosiert und aufgetragen werden kann.

Durch die Schaumbildung wird eine besonders gute, gegenüber den bisher bekannten topischen Diclofenac-Zubereitungsformen verbesserte Verteilbarkeit der erfindungsgemäßen Diclofenac-Zusammensetzungen auf der Haut erreicht, wobei zusätzlich die nichtfettende Eigenschaft des Schaumes bei der Applikation als sehr angenehm empfunden wird. Der Wirkstoff Diclofenac liegt als Salz in der Wasserphase des Schaumes gelöst vor, und ist aus diesem gut resorpierbar.

Es kann auch in der erfindungsgemäßen Zubereitung auf die Verwendung von niederen Alkoholen wie z.B. 2-Propanol verzichtet werden.

Die erfindungsgemäßen Zubereitungen sind in Druckgasbehältern abgefüllt, so daß Kontaminationen durch Mikroorganismen aus der Umwelt ausgeschlossen sind. Somit kann auf den Zusatz von Konservierungsstoffen verzichtet werden. Die Gefahr von allergischen Reaktionen oder Sensibilisierungen, die durch Konservierungsstoffe ausgelöst werden können, kann damit bei der Applikation der erfindungsgemäßen Zubereitungen vermieden werden. Hier liegt ein weiterer wesentlicher Vorteil der erfindungsgemäßen Zubereitungen.

Die nachfolgenden Beispiele sollen die Erfindung erläutern, ohne sie jedoch in ihrem Umfang zu begrenzen.

Beispiele

Als Schaum anzuwendende Diclofenac-Zubereitungen

A) Emulsion

Die Zusammensetzung der Emulsionen bestand aus folgenden Bestandteilen. Alle Angaben sind in Gew.-%.

|  | Beispiel 1 | Beispiel 2 |
|---|---|---|
| Diclofenac-Natrium | 1 | 1 |
| Natriumdodecylsulfat | 0,5 | 0,5 |
| Cetylalkohol | 1,8 | 1,8 |
| dünnflüssiges Paraffinöl | 15 | 15 |
| Polyoxyethylen(20)-sorbitan monostearat | 6 | 6 |
| Sorbitanmonostearat | 1,5 | 3,0 |
| destilliertes, keimfreies Wasser | 74,2 | 72,7 |

Diclofenac-Natrium und Natriumdodecylsulfat wurden im destillierten, keimfreien Wasser unter Rühren gelöst. Anschließend wurde die Fettphase bei 70 bis 80 ° C unter Rühren geschmolzen und das dünnflüssige Paraffinöl der Fettphase hinzugesetzt. Die Fettphase wurde unter leichtem Vakuum in die Wasserphase eingezogen und somit nach Rühren die Emulsion erhalten.

B) Emulsion enthaltende Druckgasbehälter

3

EP 0 488 089 A1

Die erhaltenen Emulsionen wurden jeweils in Druckgaspackungen abgefüllt, mit Sprühaufsätzen versehen und im Verhältnis von 45 g Emulsion mit ca. 2,5 g Treibgas beaufschlagt. Als Treibgas wurde ein Propan/Butan-Gasgemisch im Verhältnis 15:85 verwendet, dem Distickstoffoxid zur Druckeinstellung aufgedrückt wurde.

**Patentansprüche**

1. Topisch applizierbare Diclofenac-haltige Emulsionen, dadurch gekennzeichnet, daß sie 0,5 bis 3 Gew.-% eines Diclofenac-Salzes, 10 - 20 Gew.-% eines flüssigen Öles aus der Gruppe dünnflüssiger Paraffinöle und flüssiger Triglyceride von Fettsäuren mit 8 bis 18 Kohlenstoffatomen, 6 bis 14 Gew.-% eines Tensid-Gemisches aus ionischen Tensiden aus der Gruppe der Alkylsulfate und nichtionischen Tensiden aus der Gruppe der Sorbitanfettsäureester und der Gruppe der Polyoxyethylensorbitanfettsäureester und 1 bis 3 Gew.-% eines aliphatischen Alkohols mit 16 bis 18 Kohlenstoffatomen und Wasser enthalten.

2. Emulsionen nach Anspruch 1, dadurch gekennzeichnet, daß sie als Diclofenac-Salz Diclofenac-Natrium enthalten.

3. Emulsionen nach Anspruch 1 oder Anspruch 2, dadurch gekennzeichnet, daß das Tensid-Gemisch ionische und nichtionische Tenside im Verhältnis 1:10 bis 1:20 enthält.

4. Emulsionen nach Anspruch 3, dadurch gekennzeichnet, daß sie 0,25 bis 0,75 Gew.-% Natriumdodecylsulfat, 5 bis 10 Gew.-% Polyoxyethylen(20)-sorbitanmonostearat und 1,5 bis 3,0 Gew.-% Sorbitanmonostearat enthalten.

5. Emulsionen nach Anspruch 1 bis 4, dadurch gekennzeichnet, daß sie als aliphatischen Alkohol Stearylalkohol und/oder Cetylakohol enthalten.

6. Emulsionen nach Anspruch 5, dadurch gekennzeichnet, daß sie ein Gemisch aus Cetylalkohol und Stearylalkohol im Verhältnis von 1:5 bis 5:1 enthalten.

7. Diclofenac-haltige Anordnung, dadurch gekennzeichnet, daß sie die Diclofenac-haltige Emulsion gemäß Anspruch 1 bis 6 und ein Treibgas in einem mit einem Zerstäuber versehenen Druckgasbehälter angeordnet enthält.

8. Anordnung nach Anspruch 7, dadurch gekennzeichnet, daß das Treibgas Distickstoffoxid und/oder ein Propan/Butan-Gasgemisch enthält.

9. Verfahren zur Herstellung einer Diclofenac-haltigen Emulsion, dadurch gekennzeichnet, daß man eine wässrige Diclofenac-Salzlösung, enthaltend 0,5 - 3 Gew.-% Diclofenac-Salz, bezogen auf das Gesamtgewicht der Emulsion, mit 10 - 20 Gew.-% eines flüssigen Öles aus der Gruppe dünnflüssiger Paraffinöle und flüssiger Triglyceride von Fettsäuren mit 8 bis 18 Kohlenstoffatomen, 6 bis 14 Gew.-% eines Tensid-Gemisches aus ionischen Tensiden aus der Gruppe der Alkylsulfate und nichtionischen Tensiden aus der Gruppe der Sorbitanfettsäureester und der Gruppe der Polyoxyethylensorbitanfettsäureester und 1 bis 3 Gew.-% eines aliphatischen Alkohols mit 16 bis 18 Kohlenstoffatomen, jeweils bezogen auf das Gesamtgewicht der Emulsion, emulgiert.

10. Verfahren nach Anspruch 9, dadurch gekennzeichnet, daß man die erhaltene Emulsion zusammen mit einem Treibgas in einem mit einem Zerstäuber versehenen Druckgasbehälter abfüllt.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | GB-A-2 192 539 (CIBA GEIGY A G)<br>* Ansprüche 1-3 *<br>* Seite 1, Zeile 12 - Zeile 14 *<br>* Seite 1, Zeile 57 - Zeile 59 *<br>* Seite 2, Zeile 4 - Zeile 16 *<br>* Seite 2, Zeile 50 - Zeile 57 *<br>* Seite 3, Zeile 45 - Zeile 52 *<br>* Seite 3, Zeile 55 - Zeile 65 *<br>* Seite 4, Zeile 1 *<br><br>-----  | 1-10 | A61K31/195<br>A61K9/12<br>A61K47/00<br>A61K47/26<br>A61K47/20 |

**RECHERCHIERTE SACHGEBIETE (Int. Cl.5 )**

A61K

Der vorliegende Recherchenbericht wurde für alle Patentansprüche erstellt

| Recherchenort | Abschlußdatum der Recherche | Prüfer |
|---|---|---|
| DEN HAAG | 02 MAERZ 1992 | VENTURA AMAT A. |

EPO FORM 1503 03.82 (P0403)